# EUROPEAN PATENT APPLICATION

(11) **EP 0 842 650 A1**
(43) Date of publication of application: **20.05.1998**
(21) Application number: 96118500.6
(22) Date of filing: 19.11.1996
(51) Int. Cl.: A61F 13/15, A61L 15/00, B32B 5/22

(54) **Resin bonded fluid handling materials**

(71) Applicant: THE PROCTER & GAMBLE COMPANY, Cincinnati, Ohio 45202 (US)
(72) Inventor: Flohr, Andreas, 45478 Mülheim/Ruhr (DE); Bogdanski, Sabine, 61389 Schmitten, Oberreifenberg (DE); Pescher, Georg, 65812 Bad Soden (DE)
(74) Representative: Canonici, Jean-Jacques

(57) **Abstract**

The present invention is concerned with providing porous fluid handling materials which are resin bonded materials for being used as aquisition materials. The resin is impregnated into the web in a non-uniform distribution throughout the thikness of the web (i.e. the smaller Z direction perpendicular to the cross machine direction -or width- and the machine direction of the web - or length-), such a resin has a higher concentration on a weight basis on a first surface than on a the second surface. The overall, average, resin concentration is of about 25% of the basis weight, and the difference is approximated by having about two thirds of the total resin in the upper half of the web, which comrises also the first surface and is intended to be directed towards the wearer when in use.

## Description

### Field of the invention

The present invention relates to improved acquisition materials for use in disposable absorbent structures and articles, such as baby and adult incontinence diapers. In addition to improved performance, further benefits arise in the logistics area, and in the disposability area.

### Background / Prior art

Resilient structures for fluid handling webs are well known.
In particular nonwoven structures have been broadly described for being used for enhancing acquisition in absorbent structures.
A great number of presently applied materials comprise lower melting point resins, such as polypropylene, optionally comprising polyethylene.

Examples for improving such structures are described in EP-A-0.337.396 (Palumbo), e.g. an air-laid, air-through bonded web made of eccentric bi-component fibres with Polyethylene having a resin incorporated hydrophilizing agent as sheath material being combined with a polypropylene core. Whilst the fluid handling properties are improved, there is still a desire for higher resiliency, namely when being used in thin articles, which might be packed under relatively high compression forces.

A further downside of such materials is their low density before they are converted into the disposable article, resulting in requiring large volumes to be logistically handled, and stored. Additionally, during storage specific measures have to be taken so as to satisfy fire hazard requirements, resulting from the relatively high amount of air in materials with relatively high specific surface area, and relatively low flammability temperatures.

EP-A-0.312.118 (Meyer) discloses an absorbent article with a transport layer having different hydrophilicity and porosity gradient as compared to the absorbent core or the topsheet, between which the transport layer is positioned.

US-A-5.264.268 (Luceri) discloses a fibrous flow modulating layer having a pore size gradient between a flow modulating layer and the underlying fluid absorbent core. EP-A-0.397.110 (Latimer) discloses and absorbent structure comprising an improved surge management means having a basis weight of more than 60 gsm, good acquisition and rewet performance upon repeated loading. EP-A-0.672.774, EP-A-0.670.154 and US-A-5 486 166 describe heat-bonded surge management fibrous webs with improved fluid handling properties described by web properties of basis weight, void volume, permeability and porosity. Furthermore, saturation capacity and resiliency requirements are disclosed.

However, there is still a need for optimising the contradicting requirements, namely of good fluid handling both with regard to allow fast acquisition and low rewetting of the discharged fluids, which in turn is a balancing of pore size and hence resiliency and hydrophilicity / wettability, and other effects such as compatibility with polyolefinic materials, handling and disposability.

These requirements led to the development of higher resiliency materials, especially using polyester based fibres. Due to the relatively high melting point if such polymers, such webs are difficult to be heat or melt bonded, thus they are often chemically bonded by using resins, such that they are often called "resin bonded webs". Thereby the web integrity is reached by an additional chemical additive rather than melting of the fibres. Such webs do exhibit a comparatively higher density though still exhibiting good acquisition performance, primarily due to their resiliency and stiffness and hydrophilicity to allow good wetting by the fluid. Such materials also allow relative easy processing, but also carry negative effects, such as lack of compatibility with other materials of the article, namely polypropylene or polyethylene, such that bonding with such other components can become difficult, regardless whether heat fusion bonding is desired or adhesive bonding, whereby very specially selected glues need to be selected. Further, such materials do contain traceable levels of heavy metals, namely Antimony, which are undesired for disposability reasons, be this land-filling, or composting, or incineration.

### Objects of the invention

Hence it is an object of the present invention to provide resin bonded materials for being used as acquisition materials, which have improved fluid handling performance.
It is a further objective of the present invention to provide such materials without having detrimental effects on the logistic and handling system of the materials.

It is a further object of the present invention of providing such materials without detrimental effects on the after-use disposability.

Hence, the present invention is concerned with providing porous fluid handling materials which are resin bonded materials for being used as acquisition materials, which have good fluid handling performance. Preferably such materials are fibrous and produced by using the carding process.

### Brief description of drawings

Fig. 1 is showing a schematic view of an absorbent article comprising webs according to the present invention.
Fig 2 shows a the key elements of the finished product acquisition test.

### Detailed description of the invention

### Absorbent Articles

As used herein, the term "absorbent articles" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner).

An absorbent article generally comprises
- an absorbent core (which may consist of sub-structures);
- a fluid pervious topsheet;
- a fluid impervious backsheet;
- optionally further features like closure elements or elastification.

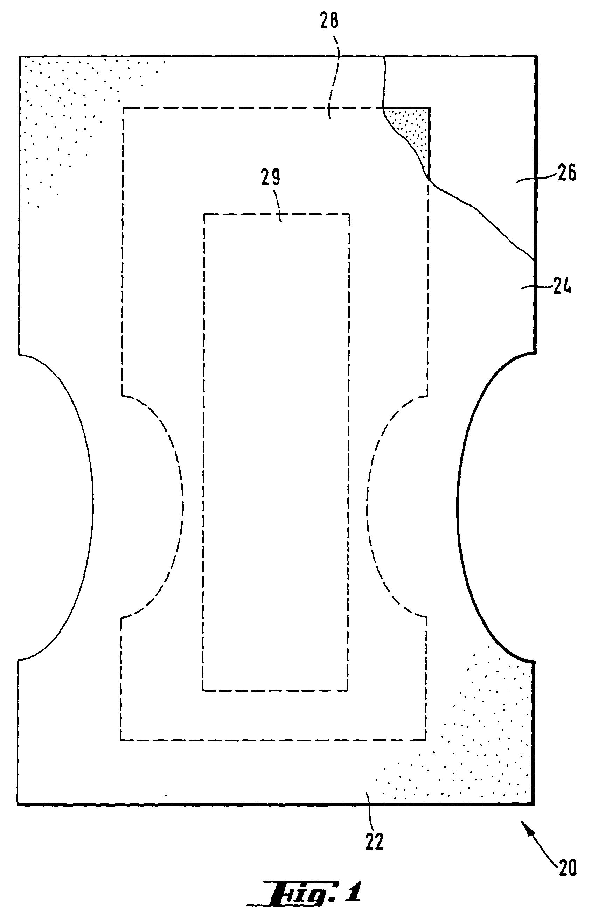

A specific embodiment of an absorbent article of the present invention is the disposable absorbent article, diaper 20, shown in Figure 1. As used herein, the term "diaper" refers to an absorbent article generally worn by infants and incontinent persons that is worn about the lower torso of the wearer. It should be understood, however, that the present invention is also applicable to other absorbent articles such as incontinent briefs, incontinent undergarments, diaper holders and liners, feminine hygiene garments, and the like.

Primarily the invention relates to the use in disposable articles with high requirements for fluid acquisition, i.e. for uses where relatively high fluid volumes at relatively high flow rates need to be absorbed, such as for disposable baby diapers, articles for severely incontinent adults, training pants and the like. However, the invention can accordingly be applied to devices with relatively lower fluid rates and volumes, such as feminine hygiene devices or articles for light or moderately incontinent adults.

Figure 1 is a plan view of the diaper 20 in its flat-out, uncontracted state (i.e. with elastic induced contraction pulled out) with portions of the structure being cut-away to more clearly show the construction of the diaper 20 and with the portion of the diaper 20 which faces or contacts the wearer, the inner surface, oriented towards the viewer. As shown in Figure 1, the diaper 20 preferably comprises a liquid pervious topsheet 24; a liquid impervious backsheet 26 joined with the topsheet 24; an absorbent core 28 positioned between the topsheet 24 and the backsheet 26; and a fluid handling material 29 positioned between the topsheet 24 and the absorbent core 28. If not specified differently, the term 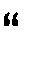 upper 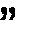 refers to the part of a structure directed towards the wearer of the article, 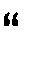 lower 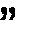 directs away from the wearer.

Figure 1 shows a preferred embodiment of the diaper 20 in which the topsheet 24 and the backsheet 26 have length and width dimensions generally larger than those of the absorbent core 28. The topsheet 24 and the backsheet 26 extend beyond the edges of the absorbent core 28 to thereby form the periphery 22 of the diaper 20. While the topsheet 24, the backsheet 26, and the absorbent core 28 may be assembled in a variety of well known configurations, preferred diaper configurations are described generally in U.S. Patent 3,860,003 entitled "Contractable Side Portions for Disposable Diaper" which issued to Kenneth B. Buell on January 14, 1975; and U.S. Patent Application Serial No. 07/715,152, allowed, "Absorbent Article With Dynamic Elastic Waist Feature Having A Predisposed Resilient Flexural Hinge", Kenneth B. Buell et al. filed June 13, 1991.

The backsheet 26 is positioned adjacent the garment surface of the absorbent core 28 and is preferably joined thereto by attachment means (not shown) such as those well known in the art. For example, the backsheet 26 may be secured to the absorbent core 28 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota and marketed as HL-1258. The attachment means will preferably comprise an open pattern network of filaments of adhesive as is disclosed in U.S. Patent 4,573,986 entitled "Disposable Waste-Containment Garment", which issued to Minetola et al. on March 4, 1986, more preferably several lines of adhesive filaments swirled into a spiral pattern such as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

The backsheet 26 is impervious to liquids (e.g., urine) and is preferably manufactured from a thin plastic film, although other flexible liquid impervious materials may also be used. As used herein, the term "flexible" refers to materials which are compliant and will readily conform to the general shape and contours of the human body. The backsheet 26 prevents the exudates absorbed and contained in the absorbent core 28 from wetting articles which contact the diaper 20 such as bed-sheets and undergarments. The backsheet 26 may thus comprise a woven or nonwoven material, polymeric films such as thermoplastic films of polyethylene or polypropylene, or composite materials such as a film-coated nonwoven material. Preferably, the backsheet is a thermoplastic film having a thickness of from about 0.012 mm to about 0.051 mm. Particularly preferred materials for the backsheet include RR8220 blown films and RR5475 cast films as manufactured by Tredegar Industries, Inc. of Terre Haute, IN, US. The backsheet 26 is preferably embossed and/or matte finished to provide a more clothlike appearance. Further, the backsheet 26 may permit vapors to escape from the absorbent core 28 (i.e., breathable) while still preventing exudates from passing through the backsheet 26.

The topsheet 24 is positioned adjacent the body surface of the absorbent core 28 and is preferably joined thereto and to the backsheet 26 by attachment means (not shown) such as those well known in the art. Suitable attachment means are described with respect to joining the backsheet 26 to the absorbent core 28. As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element.

Generally, the topsheet 24 is compliant, soft feeling, and non-irritating to the wearer's skin. Further, the topsheet 24 is liquid pervious permitting liquids (e.g., urine) to readily penetrate through its thickness. A suitable topsheet may be manufactured from a wide range of materials, such as porous foams; reticulated foams; apertured plastic films; or woven or nonwoven webs of natural fibres (e.g., wood or cotton fibres), synthetic fibres (e.g., polyester or polypropylene fibres), or a combination of natural and synthetic fibres. There are a number of manufacturing techniques which may be used to manufacture the topsheet 24. For example, the topsheet 24 may be a nonwoven web of fibres spunbonded, carded, wet-laid, meltblown, hydroentangled, combinations of the above, or the like.

The absorbent cores should be generally compressible, conformable, non-irritating to the wearer's skin, and capable of absorbing and retaining liquids such as urine and other certain body exudates. As shown in Figure 1, the absorbent core 28 has a garment surface ( 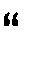 lower 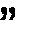 or 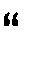 bottom 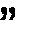 part), a body surface, side edges, and waist edges. In order to fit best into the overall absorbent article design, the absorbent core 28 may be manufactured in a wide variety of overall sizes and shapes (e.g., rectangular, hourglass, "T"-shaped, asymmetric, etc.).

The absorbent core can be made of several regions, differing in their fluid handling properties and functionality. Namely, a first region can be designed to store the fluids such that they present the minimal discomfort for the wearer, such as by firmly binding the fluids and not releasing these even under in-use pressure. A further region can be designed to transport the fluids from the acquisition region to the ultimate storage region, which can have dimensions depending on the relative arrangement of the two.

In addition, an article according to the present invention comprises an acquisition region 29. The present invention is primarily aiming at improving materials most suitable to be used in said acquisition region 29. Such a region is often being placed on top of the absorbent core 28 , i.e. between the core 28 and the topsheet 24. Such an acquisition region should have a size to at least cover the loading area of the article, i.e. the areas where the article is generally receiving the body exudates as discharge by the wearer. However, the acquisition region can be larger than the minimum, and it can have various sizes. With regard to ease of manufacturing, a preferred shape is rectangular, and a preferred size is smaller than the absorbent core 28. As indicated in Fig. 1, acquisition region 29 is designed as a "patch", i.e. having a longitudinal extension less than the absorbent core 28. In other designs, it could be preferred to have acquisition region 29 extending over the full length of the absorbent article 28.

In order to provide good acquisition functionality, the key parameter of said acquisition region 29 is fluid pick up (acquisition), which should be maximised, and fluid rewet, which should be minimised. In addition to a certain hydrophilicity requirement, the most relevant parameter of the materials used in such regions are their openness, such as expressed in density, or void volume, together with their ability to retain such an open structure even under pressure, i.e. their resiliency.

Further to these fluid handling requirements ("performance criteria") other needs have to be considered, in particular when considering that such materials can be used in mass articles such as disposable diapers, whereby large amounts of raw material have to be handled, i.e. shipped and stored, but also disposed after use.

As to the shipping and storage, it is desired to minimise the amount of "void volume", i.e. it is desired to transport a relatively higher density than is optimal for use in such applications. This, however requires a good resiliency behaviour of the web, i.e. the web should be able to regain "loft" (i.e. low density / high bulk) after being compressed strongly. Obviously, this will also provide more "loft" during use, thus allowing to maintain the high acquisition performance even under in-use pressure conditions.
Such materials can also be described by the ratio of the volume, which is taken by the solid material in the web to the total volume of the web. For ease of storage and logistics, including considerations such as requirements to address fire prevention etc., materials have preferably such a ratio of less than 10%, preferably less than 5% and even more preferably less than 4.5%.

As to the disposability, the presence of materials which might create undesired effects in public discussions should be minimised. In particular, materials containing heavy metals are not preferred, neither if the articles containing such materials are intended to be disposed in incineration units, or landfills, or composting units.

Whilst a number of well performing materials already exist, which address individual or restricted combinations of some of these requirements, and most of which belong to the class of heat-bonded materials, a simple comprehensive selection criterion does not yet exist and in particular not for chemically or resin bonded webs. More specifically, inventors have surprisingly found, that when carefully selecting the composition and making conditions of such webs, materials with improved parameter profile result.

A preferred execution of the present invention is aiming at improved properties of fibrous structures, i.e. using fibres to create acquisition materials, which are then bonded into a suitable web.

Suitable base materials to be used for the present invention have desirably a certain hydrophilicity. This, however does not mean, that these have to be completely hydrophilic (i.e. have a wetting angle for water of approximately zero), nor that this hydrophilicity has to be uniformly across all surfaces in all regions.

Generally, hydrophilicity can be achieved by starting from hydrophilic base materials, such when using cellulosic fibres, or polymeric material - which can have a varying degree of hydrophilicity. For example, polyester base materials are more hydrophilic than polypropylene based materials. In particular for the latter, a commonly used approach is to additionally use surface active materials (surfactants). When using polymeric materials, generally an extrusion step is used to form fibres, whereby such surfactants can be incorporated into the resin before these are extruded, or applied to the surface after being extruded. Alternatively, the acquisition web itself can further be hydrophilised as such, e.g. after forming a web comprising fibres made from a hydrophobic material, this can be rendered hydrophilic by addition of surfactants.

As one of the key criteria for the acquisition material properties is resiliency, the starting materials should have a certain degree of resiliency. A wide variety of resilient fibres can be envisaged to perform well in materials according to the present invention, such as well known synthetic fibres being based on polyethyleneterephatalate, polyester, polyamine, polyamides, resilient polyolefins or combinations thereof e.g. in bi-component fibre form.

Preferred fibre types are polyester (PET) based fibres and polypropylene (PP) based fibres. PET as raw material provides a particularly good resiliency, but has a less preferred disposability profile due to generally containing undesired heavy metals, like antimony, which are undesired components for example when disposing articles containing these through incineration, landfilling, or composting. PP, in contrast is broadly available, has a more preferred disposability profile, however is as raw material generally less resilient.

The constituent components of the preferred starting materials for fluid acquisition materials according to the present invention may be blended together and formed into webs by a variety of methods, including wet-laying methods, air-laying methods, carding, and other methods, of which carding methods are presently preferred.

Techniques for carding fibrous material to form sheets such as nonwovens are well known and described in the art, such as in "Nonwovens: Theory, process, performance and testing", TAPPI Press, Atlanta, GA, USA, edited by Turbak.

After having formed the web, this has to be consolidated. In contrast to a number of conventional heat-bonded webs, the acquisition materials according to the present invention comprise a further chemical additive to consolidate the fibres into a web without melt-bonding the fibre polymers. Such chemical additive binding means for increasing physical integrity of the absorbent member can be resinous binders, latex, styrene-butadiene-rubber (SBR), Ethylene-vinyl-acetate (EVA), Ethylene-vinyl- alcohols (EVOH), Acrylic based resins, and starch known in the art for providing increased integrity to fibrous webs.

Preferred chemical binders are of the Styrene-Butadiene type, such as supplied by GenCorp, Mogadore, Ohio, USA, or by BASF AG, Ludwigshafen, FRG.

In a further aspect of the present invention, it has been found, that webs being made with a resin having a high glass transition temperature (as can be determined by conventional analytical tools such as Differential Scanning Calorimetry - DSC) have improved resilience as compared to webs made with a resin having a lower one. Comparing the material properties of two webs with different T_{g} at a certain temperature (e.g. in use temperature), the resin with the higher T_{g} will be more brittle than the resin with the lower T_{g}. Particularly preferred binder materials have a T_{g} of more than 0°C, even more preferred more than 15°C. However, T_{g} should not be higher than the softening temperature of the fibre resin.

The chemical binder means - which is generally in liquid form - can be applied to the web in a number of conventional ways, generally such as running the web through a bath, or over wetted rolls, or spraying the binder onto the web.

It has been found, however, that an essential feature of the present invention is, that the web has a non-uniform application of the binder throughout its thickness. Some application techniques will provide this feature automatically (such as spraying), for some others either special steps should be taken, or special steps aiming at a homogeneous binder distribution as being desired for other applications can be omitted. The requirement of a non-even distribution is met, when about two thirds of the binder are present in about half of the web in a z-directional profile. Whilst it should be evenly distributed across the width (or cross-direction, or y-direction of the web), and the length (or machine direction or x-direction of the web), the third direction of the thickness (or z-direction) perpendicular to the other two directions should have a non-uniform distribution.
For example when examining a cross-section of such a web by means of a microscope (e.g. scanning electron microscope), it then can be visually detected that the chemical binding resin is accumulating to one of the surface. Alternative techniques, such as NMR techniques are also suitable to determine the chemical binder distribution.

The total amount of added resin is not critical for the present invention, generally a weight percentage of resin in the final, dried and cured web (i.e. excluding a carrier for the resin, which has been dried off) of between 10% and 50 % is suitable, a preferred range is between 20% and 35%, and a most preferred amount is between 25% and 10%.

After the binder is applied to the web, it has to be cured and dried. This refers to two effects, namely to provide a certain curing of the resin thus bonding both to itself but also to the fibres and also to remove water or other carrier fluid for the resin.

This can be achieved by a number of conventional techniques, generally aiming at providing energy to the web, such as running the web over heated rolls, through a heated oven, or treating it with infra-red energy beams. It is important and highly preferred, that the web is not compressed in its thickness dimension (or z-direction) during this process. This undesired effect can also occur as a side effect, such as when applying sufficient stress in length (or "machine" or x-direction of the web), which then can result in so called "neck-down" in the other perpendicular direction, namely the width (or cross-, or y-direction), but also in the z-direction. Also, when running the web for example over a number of heated rolls, certain compression of the web is required to transport it at sufficiently high speeds. Therefore, the often called "can-drying" process is not preferred, a simple drying oven, however, can be very suitable.

After the web is formed according to the above description, it can be put on rolls, and shipped to the place where it is intended to be used, e.g. for being converted into an absorbent article as described above. For transporting and logistic handling, the web can preferably be compressed heavily, thus minimising the volume to be transported. However, if the resiliency of the material is not sufficiently strong, the material might loose its performance benefits, which to an important degree depend on the openness of the structures.

A further benefit of the webs according to the present invention concerns their properties with regard to logistic and handling requirements. It is well known, that materials containing high amounts of air and a relatively large specific surface area, are more prone to fire incidents than denser and more solids containing structures. Henceforth, a preferred web according to the present invention has a volume-of-solids-to-total-volume ratio of not more than 10%, more preferably less than 5% or even more preferred less than 4.5%.

A specific aspect of the present invention relates to the basis weight of the acquisition material in the absorbent article, and how this can be best achieved. At present, carding technologies are difficult to operate, if the basis weight of the web exceeds about 40 gsm (including the binder resin weight), as the incremental basis weight per each card is limited and also the number of cards per production line should not be excessive for required space but also required effort to install the equipment. However, performance requirements of absorbent article designs often require higher basis weights than this. Thus a conventional technique is to combine two webs of relatively low basis weight onto each other. For example, this can be done at the site of the making of the web, whereby two rolls are simply unwound and combined such that the two webs have the same orientation, e.g. both webs have a the surface upwards, which belongs to that side of the web which contains the higher content of binder. Then, such conventional webs can be cut at the desired width, whereby an edge fusion process can be applied for better web handling properties.

However, it has been found, that - if the most preferred execution of running a single ply high basis weight web is not possible, the combination of lower basis weight webs into a two ply web should be done such that the two surfaces of the higher binder concentration webs are positioned outwardly, such as can most easily be achieved by folding a web along its length (x-) direction, such that the two surfaces of the lower binder concentration are positioned on top and in direct contact to each other.

*This resin bonding is achieved by a chemical binder such as of the Styrene Butadiene type of resin, which is more hydrophilic than the base polymer. It is impregnated into the web in a non-uniform distribution throughout the thickness of the web (i.e. the smaller z-direction perpendicular to the cross machine direction - or width - and the machine direction of the web - or length), such that the resin has a higher concentration on a weight basis on a first surface than on the second surface. With an overall (average) resin concentration of about 25% (weight basis), the difference can be approximated by having about two thirds of the total resin in the upper half of the web, which comprises also the first surface.*

The non-isotropic resin application also results in a different fuzziness of the surfaces, namely that the first surface with the relatively higher amount of resin is less fuzzier than the opposite side. This fuzziness difference not only results in different texturing such as can be readily determined by comparing the hand of the surfaces, *or by comparing the respective coefficient of friction values, as can be measured by well known standard methods.*

A preferred material according to the present invention has been produced by Stearns Inc., Cincinnati, Ohio, US under the trade designation 53156A, a resin bonded PET web, and listed under sample 1 in table 1. *This resin bonding is achieved by a Styrene Butadiene type of resin, which is more hydrophilic than the base polymer. Such resins can be supplied such as by GenCorp, Mogadore, Ohio, USA. The resin is impregnated into the web in a non-uniform distribution throughout the* t*hickness of the web (i.e. the smaller z-direction perpendicular to the cross machine direction - or width - and the machine direction of the web - or length), such that the resin has a higher concentration on a weight basis* *on a first surface than on the second surface. The overall (average) resin concentration of about 25% (weight basis), and the difference is approximated by having about two thirds of the total resin in the upper half of the web, which comprises also the first surface and is intended to be directed towards the wearer when in use..*

Another preferred material (sample 2 of table 1) has been produced by Veratec Inc., Walpole, US as a test material on a pilot line facility, by using PET fibres. These have been laid into a web by applying conventional carding equipment at a basis weight of 55 gsm.

A further material is also a resin bonded PET web, made by PGI, FIBERTECH, Landisville, New Jersey, US, and offered under the trade designation of type 6830. Sample 4 in table in is a conventional material, whereby two piles of about 27 gsm are combined such that are arranged in the same direction and orientation, i.e. they are made by placing them on top of each other.

Sample 3 of table 1 is the same material, except that the orientation of the webs, i.e. their relative positioning with respect to each other.

Such a materials has the descriptive parameter and performance parameter as listed in Table 1.

**Table 1**

| | Sample identification | | | |
|---|---|---|---|---|
| | 1 | 2 | 3 | 4 (comp) |
| | Veratec | Stearns | FIBERTECH | |
| number of plies [-] | 1 | 1 | 2 A | 2 B |
| basis weight [g/m2] | 55 | 55 | 55 | 55 |
| density [g/cm3] | 0.042 | 0.041 | 0.086 | 0.086 |

| Resiliency -caliper loss | | | | |
|---|---|---|---|---|
| low pressure [%] | 24 | 24 | 30 | 30 |
| peak [%] | 65 | 75 | 58 | 58 |

| Raw material acquisition. | | | | |
|---|---|---|---|---|
| 1st load [sec] | n.a | 0.63 | 0.60 | 0.71 |
| 2nd load [sec] | 0.70 | 0.90 | 1.60 | 1.90 |
| 3rd load [sec] | 0.90 | 0.99 | 1.93 | 2.14 |

*This table also shows the results for the second embodiment, namely mono-layer material having a continuous resin concentration gradient throughout its thickness (i.e. the concentration decreases essentially continuously from one surface to the other) of a basis weight of at least 40 gsm. Thereby, the fluid transfer resistance between the two layers of the web are eliminated*

A further embodiment of the present invention is a criterion allowing readily the selection of an optimised resin formulation for best resiliency and hence fluid handling performance. Surprisingly, it has been found, that webs being made with a resin having a high glass transition temperature (as can be determined by conventional analytical tools such as Differential Scanning Calorimetry - DSC) have improved resilience as compared to webs made with a resin having a lower one. Comparing three webs as described in table 2 with their Tg, and their respective performance in a finished product clearly demonstrates these effects.

Thereby, a further sample (sample 5) has been included, made as sample 3 described above, except for using a different binding resin having a lower Tg. To assess the performance, these materials have been included into a finished product, which has been produced by using a conventional diaper producing line, such as being used to produce PAMPERS BabyDry, Maxi size, except for additional insertion of the acquisition material as being subject of the present description, at a size of 241 mm by 90 mm, positioned centered in CD direction an 28 mm offset the front, untaped core end, and a rebalancing of the remaining absorbency materials, namely changing the amount of crosslinked cellulose material from 5.3 g to 6.6 g, of the storage core fluff amount from 23 g to 19 g and of the superabsorbent amount from 8g to *13g*. Also, the type of superabsorbent was changed, from previously used type SXM 100 as available from STOCKHAUSEN GmbH, Krefeld, Germany, to type *T5383* of the same supplier or *type ASAP 2300 of Chemdal Ltd, Manchester, UK.*

The resulting product was then tested according to the finished product acquisition test, as described further.

**Table 2**

| | Sample identification | | |
|---|---|---|---|
| | 1 | 3 | 5 |
| | Veratec | FIBERTECH | |
| number of plies | 1 | 2 | 2 |
| Tg [°C] | +15 | -5 | -20 |

| Finished Product acquisition [ml/sec] | | | |
|---|---|---|---|
| 1st gush | 7.65 | 5.68 | 5.28 |
| 2nd gush | 5.47 | 3.18 | 2.99 |
| 3rd gush | 3.28 | 1.94 | 1.45 |
| 4th gush | 2.04 | 1.23 | 0.83 |

Clearly, the web being made by using the lower Tg resin has a better performance than the otherwise equally made web using the other resin. The web with the preferred performance is based on SBR polymers.

Whilst so far the key and performance related aspects of the present invention have been discussed, additional benefits of webs being made according to the present invention are described now.

First, in particular when applying these webs according to the present invention in disposable absorbent articles, the fate of the various by-components is relevant. It has been found, that PET fibre containing webs to have increased levels of undesired components, namely of Antimony. Hence it is a further object of the invention to provided webs with improved performance properties and still being preferred for not containing such elements. This can be achieved by selecting the base resins without compromising the fluid handling performance according to the present criteria.

A particularly preferred execution of such webs comprise PP fibres, which are - instead of the widely used thermal bonding - are chemically bonded, preferably with a SBR resin. As the fibre resin per se has less overall resiliency, it is more preferred to produce such webs at densities of not more than 0.055 g/cm3, whilst still having sufficient resilient power to provide good performance.

A further benefit of the webs according to the present invention concerns their properties with regard to logistic and handling requirements. It is well known, that materials containing high amounts of air and a relatively large specific surface area, are more prone to fire incidents than denser and more solids containing structures. Henceforth, it is useful to apply the teachings of the present invention particularly to webs having a density of less than *0.05g/cm3* and also having a solid-to-volume ratio of less than 10%, preferably less than 5 % more preferably less than 4.5%.

Whilst the above has been described for webs essentially made of fibrous structures, the same principles can be applied to other porous structures, such as foams or particle aggregate structures, and the like, provided they satisfy the acquisition performance criteria according to the present invention.

### Test Procedures

Unless specified otherwise, the tests are carried out under controlled laboratory conditions of about 23 +/- 2°C and at 50 +/-10% relative humidity. Test specimen are stored under these conditions for at least 24 hours before testing.

### Density / caliper / basis weight measurement

A specimen of a defined area such as by cutting with a sample cutter is weighed to at least 0.1% accuracy. Caliper is measured under an applied pressure of *550 Pa (0.08 psi)* by using a conventional caliper measurement device with a flat plate with a *diameter of 2 cm,* which can be loaded with defined weights. The test specimen can then be placed between this plate and a flat surface and the distance between the plate and the base surface can be measured. The standard caliper measurement is executed by carefully (to avoid over compression) applying *a weight of 225 g,* resulting in a pressure of 1747 Pa. The weight is left for at least about 5 seconds, upon which the distance reading is taken. This procedure is repeated at least three times for one specimen to provide a representative number of test data.

The basis weight of a test specimen can be tested by determining sufficiently accurately the weight of a test specimen of known area. Conveniently, a test specimen of 10 cm by 10 cm is weighted e.g. on a scale having an accuracy of 0.001 g.

Basis weight as weight per unit area expressed in g/m2, caliper expressed in mm at 550 Pa pressure, and density expressed in g/cm3 can be readily calculated.

### Resiliency measurement

The resiliency measurement is performed on a stack of sample material by compressing and decompressing it in a dynamometer, such as an Instron instrument.

Samples are prepared at a 4 cm by 4 cm size. A stack of 15 plies of sample material (approximately 3 cm high when uncompressed) is placed between two metal plates, which are larger than the sample size, and which are mounted in a conventional compression/ pressure analysis equipment, such as an INSTRON tester. The instrument is then operated at a crosshead speed of *100 mm per minute* in compression and decompression cycles and the displacement as well as corresponding force values (which translate into respective pressures) are recorded, such as by graphical presentation or in a data file in a connected or internal computer unit.

The equipment is operated in three cycles each between no pressure and up to 4826 Pa (0.7psi). The corresponding thickness of the 15 piles is noted.

The Thickness loss at peak stress (4826 Pa) and Thickness loss after stress (69 Pa {0.01 psi}) is recorded by averaging the values as taken from the three cycles, and the relating to the *initial caliper as measured according to the method describe before.*

### Synthetic urine formulation.

Unless specified explicitly, the specific synthetic urine used in the test methods is commonly known as Jayco SynUrine and is available from Jayco Pharmaceuticals Company of Camp Hill, Pennsylvania. The formula for the synthetic urine is: 2.0 g/: of KCl; 2.0 g/l of Na2SO4; 0.85 g/l of (NH4)H2PO4; 0.15 g/l (NH4)H2PO4; 0.19 g/l of CaCl2; ad 0.23 g/l of MgCl2. All of the chemicals are of reagent grade. The pH of the synthetic Urine is in the range of 6.0 to 6.4.

### Raw Material Acquisition test

In order to assess the performance properties of the raw material, a standard industry test method has been modified to allow better distinction with regard to the fluid handling characteristics. The starting point is EDANA Recommended Test 150.3-96: Nonwoven coverstock Strike Through Time, as published by the European Disposables and Non-wovens Association (EDANA), Brussels, Belgium, February 1996. Essentially, this method measures the time a known amount of test fluid needs to penetrate the test specimen. The test set up comprises a specific test plate with a defined orifice and an electric timer connected to electrodes at located in the orifice

In order to demonstrate the benefits of the present invention, this test has been modified as follows: First, the test fluid has been replaced by the one described herein above. Second, the test has been repeated three times, with a waiting time of one minute between the loads on the same test pads underlying the specimen, and the three respective strike-trough times have been recorded.

### Finished-Product-Acquisition Test

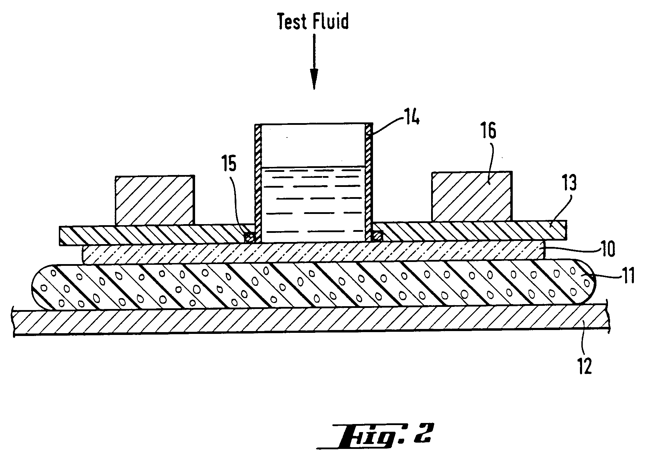

Referring to Figure 2, an absorbent structure (10) is loaded with a 75 ml gush of synthetic urine at a rate of 15 ml/s using a pump (Model 7520-00, supplied by Cole Parmer Instruments., Chicago, U.S.A.), from a height of 5 cm above the sample surface. The time to absorb the urine is recorded by a timer. The gush is repeated every 5 minutes at precisely 5 minute gush intervals until the article is sufficiently loaded. Current test data are generated by loading four times.

The test sample, which comprises a core and includes a topsheet and a backsheet, is arranged to lie flat on a foam platform 11 within a perspex box (only base 12 of which is shown). A perspex plate 13 having a 5 cm diameter opening substantially in its middle is placed on top of the sample. Synthetic urine is introduced to the sample through a cylinder 14 fitted, and glued into the opening. Electrodes 15 are located on the lowest surface of the plate, in contact with the surface of the absorbent structure 10. The electrodes are connected to the timer. Loads 16 are placed on top of the plate to simulate, for example a baby's weight. A pressure of 50g/cm2 (0.7psi) is typically utilised in this test.

As test fluid is introduced into the cylinder it typically builds up on top of the absorbent structure thereby completing an electrical circuit between the electrodes. This starts the timer. The timer is stopped when the absorbent structure has absorbed the gush of urine, and the electrical contact between the electrodes is broken.

The acquisition rate is defined as the gush volume absorbed (ml) per unit time (s). The acquisition rate is calculated for each gush introduced into the sample. Of particular interest in view of the current invention are the first and the last of the four gushes.

This test is primarily designed to evaluate products having an absorbent capacity of about 300 ml to 400 ml. If products with significantly different capacities should be evaluated, the settings in particular of the fluid volume per gush should be adjusted appropriately to about 20% of the theoretical capacity, and the deviations should be recorded.

## Claims

**1.** A porous fluid handling material for use in an absorbent article, having a first and a second surface extending in essentially planar directions perpendicular to each other (x-, y- directions), and a thickness (z-direction) extending perpendicularly to the other two directions,
comprising a base polymer material,
said fluid handling material having a density of less than 0.15g/cm3 @ 1747 Pa pressure,
and being chemically bonded
with a resin
which is more hydrophilic than the base polymer;
and which is non-uniformly distributed in said fluid handling material throughout the thickness (z-) direction,
such that at least two thirds of the resin are contained in the upper half of the material which is oriented towards the wearer when in use,
thereby having a higher concentration of resin on a weight basis on the first surface than on the second surface,
characterised in that said fluid handling material
has a raw material acquisition performance of less than 2 seconds at the third gush.

**2.** A porous fluid handling material according to claim 1,
further characterised in that it has a raw material acquisition performance of less than 1 second at the third gush.

**3.** A porous fluid handling material according to claims 1 or 2, further characterised in that
it has a basis weight of more than 40 gsm,
and is made of a one layer material .

**4.** A porous fluid handling material according to claims 1 to 3, further characterised in that
it has a basis weight of more than 40 gsm,
resulting from folding a one-layer material such that said two second surfaces which have a lower resin concentration are in direct contact with each other.

**5.** A porous fluid handling material according to claim 4, further characterised in that
it has a basis weight of more than 50 gsm,
resulting from folding a one-layer material such that said two second surfaces which have a lower resin concentration are in direct contact with each other.

**6.** A porous fluid handling web/substrate made according to claim 3, further characterised in that
it retains more than 75% of its caliper when measured according to the described resiliency testing.

**7.** A porous fluid handling material according to claim 1, further characterised in that
the bonding resin has a glass transition temperature T_{g} of more than 5°C.

**8.** A porous fluid handling material according to claim 7,
with T_{g} being higher than 15°C.

**9.** A porous fluid handling material according to claim 7
further characterised in that
with resin comprising polymers selected from the group of styrene-butadiene-rubber (SBR), Ethylene-vinyl-acetate (EVA), Ethylene-vinyl- alcohols (EVOH), Acrylic based resins,

**10.** A porous fluid handling material according to claim 1, further characterised in that
it comprises fibres.

**11.** A porous fluid handling material according to claim 9, further characterised in that
said fibres comprise polymers selected from the group of polyolefins, polyester, polyamines, polyamides.

**12.** A porous fluid handling material according to claim 9, further characterised in that
said fibres comprise polypropylene.

**13.** A porous fluid handling material according to claim 10, further characterised in that
the web is essentially Antimony free.

**13.** A porous fluid handling material according to claim 11 with a density of less than 0.055 g/cm3

**14.** A porous fluid handling material according to claim 1 with a resiliency of more than 75% .

**15.** A porous fluid handling material according to claim 11, whereby the material is fibrous and has been formed by means of a carding process.

**16.** A porous fluid handling material according to claim 13 further characterised in that
it has a value of less than 10% by weight of solids to volume.

**17.** A porous fluid handling material according to claim 13 further characterised in that
it has a value of less than 5% by weight of solids to volume.

**18.** A porous fluid handling material according to claim 13 further characterised in that
it has a value of less than 4.5% by weight of solids to volume.

**19.** An absorbent hygienic article comprising a porous fluid handling material according to any of the preceding claims.

**20.** A method of producing a porous fluid handling layer by
forming a carded web having a basis weight of more than 20 gsm;
selecting a chemical binder resin laving a glass transition temperature Tg of less than 5°C;
impregnating said web with said resin such that the resin is distributed non-uniformly throughout the thickness of the web thereby creating one surface of the web having a higher resin content than the opposite side of the web.

**21.** A method according to claim 20, further comprising the step of submitting said resin impregnated web to increased temperatures for curing and drying whilst it is essentially in an uncompressed state.

**22.** A method according to claim 20, further characterised in that
the carded web is formed at a basis weight of more than 40 gsm.

**23.** A method for producing an absorbent hygienic article comprising the step of
using a porous fluid handling layer according to claim 5, whereby the folding of the web is performed as a process step of the making the article.

**24.** Use of a porous fluid handling material according to any of claims 1 to 18 in a hygienic absorbent article having a finished product acquisition performance of more than 1.5 ml/second in the fourth gush.

**25.** Use of a chemical binder selected from the group of styrene-butadiene-rubber (SBR), Ethylene-vinyl-acetate (EVA), Ethylene-vinyl- alcohols (EVOH), Acrylic based resins to bind a porous fluid handling material comprising fibres selected from the group of polyolefins having a density of less than 0.055 g/ cm3.

**26.** Use of an drying oven allowing essentially untensioned conditions during drying to produce a material according to any of claims 1 to 18.

**27.** Use of chemical binder resins to produce a porous fluid handling material comprising polyolefinic fibres.

**28.** Use of a porous fluid handling materials according to any of the preceding claims in a disposable absorbent article.
